# EUROPEAN PATENT SPECIFICATION

(11) **EP 2 065 060 B1**
(45) Date of publication and mention of the grant of the patent: **27.10.2010**
(21) Application number: 07381073.1
(22) Date of filing: 30.11.2007
(51) Int. Cl.: A61L 9/12, B01D 53/26, B01D 53/04

(54) **Air freshener anti-humidity tablet**
Anti-Feuchtigkeits-Lufterfrischungstablette
Pastille anti-humidité rafraîchissant l'air

(43) Date of publication of application: 03.06.2009
(73) Proprietor: Top Grade, S.L., 43004 Tarragona (ES)
(72) Inventor: Schmidt Dios, Eva, 43110 La Canonja (ES)
(74) Representative: Martin Santos, Victoria Sofia

(56) References cited:
- WO-A-99/16536
- DE-A1- 10 354 037
- US-A1- 2003 024 997
- US-A1- 2005 118 055

## Description

### OBJECT OF THE INVENTION

The present invention refers to an air freshener anti-humidity tablet. The tablet comprises an agent for drying ambient humidity and, when necessary, is also used to absorb ambient humidity, to provide an agreeable aroma. It is designed mainly for use in closed environments of average dimensions such as, for example, rooms.

The tablet which is the object of the invention comprises a drying material and is **characterised in that** it is provided with at least one air freshener device which comprises a container which has part of its surface in contact with the tablet, and part of its surface in direct contact with the atmosphere, with the contact surface of the tablet being impermeable and the surface in contact with the atmosphere being provided with a permeable membrane which releases a freshening agent from inside the container.

In this way it is possible to combine in the same element an anti- humidity function and an air freshening function, with a means which provides a gradual release into the atmosphere of the fragrance.

### BACKGROUND TO THE INVENTION

Compacted means of humidity absorption using absorbent agents are well known, such as, for example those in the form of small granules or in larger tablets. These agents act by absorbing humidity and transforming it into water, which is then stored in a collector situated underneath. When the drying agent has been consumed, the liquid is emptied and a replacement is fitted.

Damp areas frequently generate bad odours. Therefore, some of the aforementioned humidity absorption or drying agents contain additives which release a pleasant fragrance, thus achieving a reduction in unpleasant odours.

However, these means include in their composition aromatic additives which have the disadvantage that their fragrance is rapidly released so that it is consumed before the absorption capacity of the anti-humidity has been exhausted.

Permeable membranes are also known which regulate the discharge over time of a liquid into the atmosphere.

The present invention resolves the previous disadvantage by means of a tablet which is provided with the means for controlling the rate at which the fragrance is released. In this way the rate of release of the vapours is considerably reduced, achieving an improved spread of the evaporation of the freshening agent throughout the durable life of the drying product.

It is known in the state of the art chemical compositions and methods for absorbing moisture as the one disclosed in US 2005118055 wherein is taught a method for absorbing moisture and odour from a chamber which is achieved by addition to the chamber of a desiccant composition comprising a substantially water-insoluble polyacrilate compound capable of absorbing at least its own weight of moisture.

It is also known from the state of the art another freshener compositions articles as the one disclosed in US 2003024997 wherein is shown air freshening composition including porous carrier particles having a perfume trapping therein and a second component selected from an inert filer, hygroscopic agent, binder, coating material, moisture providing agent and mixtures thereof,

It is also known from the state of the art document WO 02089862 which discloses a package containing an air freshening composition wherein the package comprises a container which has an opening and a reclosable lid over the opening wherein the lid may be opened to allow moisture to enter the container and contact the air freshening article. The lid comprises a permeable membrane and a resealable cover for covering the membrane. The membrane made from cloth, wire mesh, and permeable and semi-permeable films that will allow fluid communication therethrough.

### DESCRIPTION OF THE INVENTION

The present invention refers to a tablet which absorbs atmospheric humidity while at the same time providing a pleasant fragrance. The tablet includes a drying agent for the absorption of of humidity. This agent could be, for example, calcium chloride which is compacted in tablet form and which may be given any shape.

Its basic application is the absorption of humidity and the freshener of small, medium or large sized closed areas.

The tablet which is the object of this invention is **characterised in that** it is provided with at least one air freshener device comprising a container which is provided with part of its surface in contact with the tablet and part of its surface in direct contact with the atmosphere, with the surface in contact with the atmosphere in possession of a permeable membrane which releases a fragrance agent in the container.

In this way both the drying and the freshener functions are combined, with the latter function carried out throughout the full useful life of the drying product. The tablet which is the object of this invention combines in a single element both functions through a means which provides a gradual release into the atmosphere of the fragrance, and which also enables the elimination of freshener additives from the composition of the anti-humidity tablet.

### DESCRIPTION OF THE DRAWINGS

The present descriptive report is accompanied by a set of plans, which are illustrative of a preferred embodiment but which are in no way restrictive of the invention.

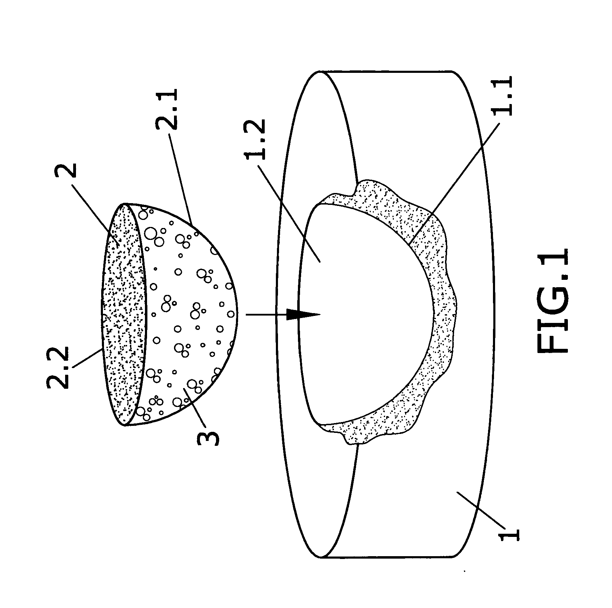

Figure 1 shows a perspective diagram of a preferred embodiment of the invention.

### PREFERRED EMBODIMENT OF THE INVENTION

Figure 1 shows a preferred embodiment of the tablet (1) which is the object of the invention.

The freshener device (2) is provided with impermeable walls 2.1) in contact with the tablet, so that the liquid content (3) of the freshener device (2) cannot migrate to the tablet, thus preventing any loss of the properties of the fragrance. The device (2) is provided with a wall, in this case in its upper part (2.2) which is in contact with the atmosphere. This wall (2.2) is provided with a permeable membrane which releases the freshener agent (3) through said membrane.

In the preferred embodiment shown, the table is provided with a cavity (1.1) which has the same shape as the wall (2.1) in contact with the tablet of the freshener element (2) and into which the tablet fits.

The freshener device (2) and the tablet (1) may be connected by various means, such as, for example, an adhesive.

## Claims

1. Anti-humidity tablet comprising a drying material **characterised in that** it is provided with at least one freshener device (2) having
(i) impermeable walls (2.1) in contact with the tablet (1), so that the liquid content (3) of the freshener device (2) cannot migrate to the tablet, thus preventing any loss of the properties of the fragrance, and
(ii) a wall in contact with the atmosphere and provided with a permeable membrane which releases the freshener agent (3) through said membrane.

2. Anti-humidity tablet according to claim 1, **characterised in that** it is provided with a cavity (1.1) into which the freshening device (2) fits.

3. Anti-humidity tablet according to claim 2, **characterised in that** the freshener device (2) and the tablet (1) are connected by means of an adhesive.

## Patentansprüche

1. Antifeuchtigkeitstablette, die ein Trocknungsmaterial umfasst, **dadurch gekennzeichnet, dass** sie mit wenigstens einer Erfrischungsvorrichtung (2) versehen ist, die
(i) mit der Tablette (1) in Kontakt stehende undurchlässige Wände (2.1) hat, so dass der flüssige Inhalt (3) der Erfrischungsvorrichtung (2) nicht in die Tablette wandern kann, wodurch jeglicher Verlust der Eigenschaften des Duftes verhindert wird, und
(ii) eine mit der Umgebungsluft in Kontakt stehende und mit einer durchlässigen Membran versehene Wand, die den Erfrischungswirkstoff (3) durch die besagte Membran freisetzt.

2. Antifeuchtigkeitstablette nach Anspruch 1, **dadurch gekennzeichnet, dass** sie mit einer Vertiefung (1.1) versehen ist, in die die Erfrischungsvorrichtung (2) passt.

3. Antifeuchtigkeitstablette nach Anspruch 2, **dadurch gekennzeichnet, dass** die Erfrischungsvorrichtung (2) und die Tablette (1) mittels eines Klebers verbunden sind.

## Revendications

1. Pastille antihumidité comprenant un matériel de séchage, **caractérisée en ce qu'**elle est munie d'au moins un dispositif désodorisante (2) ayant.
(i) des parois imperméables (2.1) en contact avec la pastille (1), de manière à ce que le contenu liquide (3) du dispositif désodorisante (2) ne puisse pas migrer vers la pastille, en évitant ainsi toute perdre des propriétés du parfum, et
(ii) une paroi en contact avec l'atmosphère et munie d'une membrane perméable qui libère l'agent désodorisante (3) à travers de ladite membrane.

2. Pastille antihumidité selon la revendication 1, **caractérisée en ce qu'**elle est munie d'une cavité (1.1) dans laquelle le dispositif désodorisante (2) s'emboîte.

3. Pastille antihumidité selon la revendication 2, **caractérisée en ce que** le dispositif désodorisante (2) et la pastille (1) sont reliés au moyen d'un adhésif.
